(19) Europäisches Patentamt
European Patent Office
Office européen des brevets

(11) Veröffentlichungsnummer: **0 622 363 A2**

(12) # EUROPÄISCHE PATENTANMELDUNG

(21) Anmeldenummer: **94105772.1**

(22) Anmeldetag: **14.04.94**

(51) Int. Cl.[5]: **C07D 401/04**, A61K 31/445

(30) Priorität: **27.04.93 DE 4313695**

(43) Veröffentlichungstag der Anmeldung:
**02.11.94 Patentblatt 94/44**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Stoltefuss, Jürgen, Dipl.-Ing.**
**Parkstrasse 20**
**D-42781 Haan (DE)**
Erfinder: **Goldmann, Siegfried, Dr.**
**Am Osterholz 91**
**D-42327 Wuppertal (DE)**
Erfinder: **Straub, Alexander, Dr.**
**Moospfad 30**
**D-42113 Wuppertal (DE)**
Erfinder: **Bechem, Martin, Dr.**
**Hans-Böckler-Strasse 102**
**D-42111 Wuppertal (DE)**
Erfinder: **Gross, Rainer, Prof. Dr.**
**Platzhofstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul, Dr.**
**Pahlkestrasse 15**
**D-42115 Wuppertal (DE)**

(54) **2-Amino-5-cyano-4-chinolyldihydropyridinester, Verfahren zu ihrer Herstellung und ihre Verwendung in Arzneimitteln zur Behandlung von Herz-Kreislaufkrankungen.**

(57) Die vorliegende Erfindung betrifft neue 2-Amino-5-cyano-4-chinolyl-dihydropyridinester der allgemeinen Formel (I)

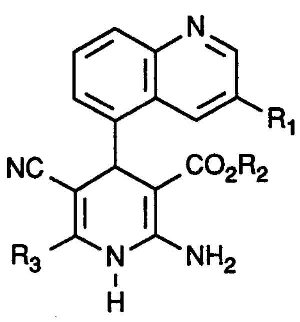

(I)

in welcher $R_1$ bis $R_3$ die in der Beschreibung angegebene Bedeutung haben, Verfahren zu ihrer Herstellung und ihre Verwendung in Arzneimitteln, insbesondere in Mitteln zur Behandlung von Herz-Kreislauferkrankungen.

EP 0 622 363 A2

Die vorliegende Erfindung betrifft neue 2-Amino-5-cyano-4-chinolyl-1,4-dihydropyridinester, Verfahren zu ihrer Herstellung und ihre Verwendung in Arzneimitteln, insbesondere in Mitteln zur Behandlung von Herz-Kreislauferkrankungen.

Es ist bereits bekannt, daß einige 2- und 6-Amino-3,4-dihydropyridine neben einer antiarrhythmischen auch eine lipidabsorptionshemmende Wirkung besitzen. Auch 2-Amino-1,4-dihydropyridine mit einer vasodilatorischen und antihypertensiven Wirkung sind schon beschrieben.

Einige der erfindungsgemäßen Verbindungen der Formel (I) werden von den allgemeinen breiten Beschreibung in EP 71 819 umfaßt, ohne dort jedoch konkrete genannt zu sein. In EP A 515 940 werden auch 2-Amino-5-cyano-4-chinolyl-dihydropyridine beschrieben, die eine positiv inotrope Wirkung besitzen.

Die vorliegende Erfindung betrifft neue 2-Amino-5-cyano-4-chinolyl-dihydropyridinester der allgemeinen Formel (I)

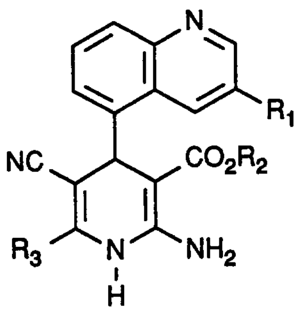

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Hydroxy, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel $-NR^4R^5$ substituiert ist,
worin

$R^4$ und $R^5$ gleich oder verschieden sind und Wasserstoff, geradkettiges oder verzweigtes Alkyl mit bis zu 8 Kohlenstoffatomen, Phenyl oder Benzyl bedeuten,

oder

für Pyridyl oder Thienyl steht, die gegebenenfalls durch Halogen substituiert sind,

$R^2$ für einen geradkettigen, verzweigten, cyclischen, gesättigten oder ungesättigten Kohlenwasserstoffrest mit bis zu 12 Kohlenstoffatomen steht, der immer 1- oder 2-fach gleich oder verschieden substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$
worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3, 4 oder 5 bedeutet,

d eine Zahl 0, 1, 2, 3, 4 oder 5 bedeutet,

c die unten angegebene Bedeutung von a hat und mit dieser gleich oder verschieden ist,

$R^{15}$ und $R^{16}$ gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substitu-

iert ist,

$R^{19}$ und $R^{20}$ gleich oder verschieden sind und geradkettiges oder verzweigtes Alkyl mit 2 bis 8 Kohlenstoffatomen bedeuten, das durch Halogen, Hydroxy oder Phenyl substituiert ist, das seinerseits durch Halogen, Nitro, Hydroxy oder durch geradkettiges oder verzweigtes Alkyl oder Alkoxy mit jeweils bis zu 4 Kohlenstoffatomen substituiert sein kann, oder
Cycloalkyl mit 3 bis 8 Kohlenstoffatomen bedeuten, oder
geradkettiges oder verzweigtes Alkenyl mit 3 bis 8 Kohlenstoffatomen bedeuten, oder
Phenyl bedeuten, das durch Halogen, Nitro oder geradkettiges oder
verzweigtes Alkyl oder Alkoxy mit bis zu 4 Kohlenstoffatomen substituiert ist,
oder

$R^{19}$ und $R^{20}$ gemeinsam mit dem Stickstoffatom einen 4- bis 7-gliedrigen Heterocyclus bilden, der gegebenenfalls durch ein Sauerstoff- oder Schwefelatom oder durch einen Rest der Formel $-NR^{21}$ unterbrochen ist,
worin

$R^{21}$ Wasserstoff oder geradkettiges oder verzweigtes Alkyl mit bis zu 8 Kohlenstoffatomen bedeutet, das gegebenenfalls durch Phenyl substituiert ist, das seinerseits durch Halogen substituiert sein kann, oder
Phenyl bedeutet, das gegebenenfalls durch Halogen substituiert ist,

oder substituiert ist durch einen 3- bis 7-gliedrigen gesättigten oder ungesättigten Heterocyclus oder Heterocyclyloxyring mit bis zu 3 Heteroatomen aus der Reihe S, N oder O, der seinerseits bis zu 2-fach gleich oder verschieden durch Halogen, Hydroxy oder durch Aryl oder Arylsulfonyl mit 6 bis 10 Kohlenstoffatomen substituiert sein kann, wobei die Cyclen ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,

oder der Heterocyclus gegebenenfalls bis zu 2-fach gleich oder verschieden durch geradkettiges oder verzweigtes Alkyl oder Alkenyl mit jeweils bis zu 8 Kohlenstoffatomen substituiert ist, die ihrerseits bis zu 2-fach gleich oder verschieden durch $-NR^{22}R^{23}$ substituiert sein können,

worin

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, das gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können, oder
Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,
oder

$R^{22}$ und $R^{23}$ gemeinsam mit dem Stickstoffatom einen 5- bis 7-gliedrigen, gesättigen oder ungesättigten Heterocyclus mit bis zu 3 Heteroatomen aus der Reihe S, N oder O bilden,

oder Alkyl oder Alkenyl ihrerseits durch Phenyl oder Phenoxy substituiert sein können, die gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sind,

der gegebenenfalls durch ein Sauerstoffatom oder durch Aryliden mit 6 bis 10

Kohlenstoffatomen unterbrochen sein kann, das gegebenenfalls durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,
oder der gegebenenfalls durch eine Gruppe der Formel $-S(O)_a$ oder $-NR^6$ unterbrochen ist,
worin

a eine Zahl 0,1 oder 2 bedeutet
und

$R^6$ Wasserstoff, Aryl mit 6 bis 10 Kohlenstoffatomen oder geradkettiges oder verzweigtes Alkyl mit bis zu 6 Kohlenstoffatomen oder Cycloalkyl mit 3 bis 8 Kohlenstoffatomen bedeutet, wobei Alkyl und Cycloalkyl gegebenenfalls durch Aryl mit 6 bis 10 Kohlenstoffatomen substituiert sind

und wobei der Kohlenwasserstoffrest gegebenenfalls zusätzlich durch Phenyl substituiert ist, das seinerseits durch Halogen substituiert sein kann,
und

$R^3$ für Wasserstoff oder für geradkettiges oder verzweigtes Alkyl mit bis zu 8 Kohlenstoffatomen steht,

und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher

$R^1$ für Phenyl steht, das gegebenenfalls bis zu 2-fach gleich oder verschieden durch Halogen, Nitro, Cyano, Hydroxy, Trifluormethyl oder durch geradkettiges oder verzweigtes Alkyl oder Alkoxy mit jeweils bis zu 4 Kohlenstoffatomen oder durch eine Gruppe der Formel $-NR^4R^5$ substituiert ist,
worin

$R^4$ und $R^5$ gleich oder verschieden sind und Wasserstoff, geradkettiges oder verzweigtes Alkyl mit bis zu 6 Kohlenstoffatomen, Phenyl oder Benzyl bedeuten,

oder für Pyridyl oder Thienyl steht, die gegebenenfalls durch Fluor, Chlor oder Brom substituiert sind,

$R^2$ für einen geradkettigen, verzweigten, cyclischen, gesättigten oder ungesättigten Kohlenwasserstoffrest mit bis zu 10 Kohlenstoffatomen steht, der immer 1- oder 2-fach gleich oder verschieden substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$
worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind

b eine Zahl 1, 2, 3 oder 4 bedeutet,

d eine Zahl 0, 1, 2, 3 oder 4 bedeutet,

c die unten angegebene Bedeutung von a hat und mit

dieser gleich oder verschieden ist,

$R^{15}$ und $R^{16}$ gleich oder verschieden sind und Phenyl bedeuten, das gegebenenfalls bis zu 2-fach gleich oder verschieden durch Fluor, Chlor, Nitro, Carboxy oder durch geradkettiges oder verzweigtes Alkyl, Alkoxy oder Alkoxycarbonyl mit jeweils bis zu 4 Kohlenstoffatomen substituiert ist,

$R^{19}$ und $R^{20}$ gleich oder verschieden sind und geradkettiges oder verzweigtes Alkyl mit 2 bis 8 Kohlenstoffatomen bedeuten, das durch Fluor, Chlor, Brom oder Phenyl substituiert ist, das seinerseits durch Fluor, Chlor, Brom, Methyl, Ethyl, Methoxy oder Ethoxy substituiert sein kann, oder

Cyclopropyl, Cyclopentyl oder Cyclohexyl bedeuten, oder

Phenyl bedeuten, das durch Fluor, Chlor, Brom, Nitro, Methyl, Ethyl, Methoxy oder Ethoxy substituiert ist,

oder

$R^{19}$ und $R^{20}$ gemeinsam mit dem Stickstoffatom einen 5- bis 7-gliedrigen Heterocyclus bilden, der gegebenenfalls durch ein Sauerstoff- oder Schwefelatom oder durch einen Rest der Formel $-NR^{21}$ unterbrochen ist,

worin

$R^{21}$ Wasserstoff oder geradkettiges oder verzweigtes Alkyl mit bis zu 10 Kohlenstoffatomen bedeutet, das gegebenenfalls durch Phenyl substituiert ist, das seinerseits durch Fluor, Chlor oder Brom substituiert sein kann, oder

Phenyl bedeutet, das gegebenenfalls durch Fluor, Chlor oder Brom substituiert ist,

oder substituiert ist durch Pyridyl, Tetrahydropyranyl, Pyrazolyl, Furyl, Chromanyl, Piperazinyl, Piperidinyl, Isochinolidinyl oder durch einen Rest der Formel

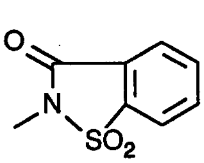 , 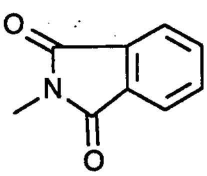 oder 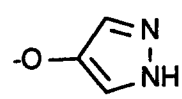

die ihrerseits durch Fluor, Chlor, Benzyl, Phenyl oder Phenylsulfonyl substituiert sein können,

und wobei der Kohlenwasserstoffrest ($R^2$) gegebenenfalls zusätzlich durch Sauerstoff, durch Phenyliden oder durch $-S(O)_a$ oder $-NR^6$ unterbrochen ist,

worin

a eine Zahl 0, 1 oder 2 bedeutet

und

$R^6$ Wasserstoff, Phenyl oder geradkettiges oder verzweigtes Alkyl mit bis zu 5 Kohlenstoffatomen, Cyclopropyl, Cyclopentyl oder Cyclohexyl bedeutet, die gegebenenfalls durch Phenyl substituiert sind

und wobei der Kohlenwasserstoffrest gegebenenfalls durch Phenyl substituiert ist, das seinerseits durch Fluor, Chlor oder Brom substituiert sein kann,

$R^3$ für Wasserstoff oder für geradkettiges oder verzweigtes Alkyl mit bis zu 4 Kohlenstoffatomen steht

und deren Salze.

Besonders bevorzugt sind Verbindungen der allgemeinen Formel (I),

in welcher

$R^1$ für Phenyl steht, das gegebenenfalls bis zu 2-fach

gleich oder verschieden durch Fluor, Chlor, Nitro, Cyano, Hydroxy, Trifluormethyl, Methyl, Ethyl, Methoxy oder Ethoxy oder durch eine Gruppe der Formel $-NR^4R^5$ substituiert ist,
worin

$R^4$ und $R^5$ gleich oder verschieden sind und Wasserstoff oder geradkettiges oder verzweigtes Alkyl mit bis zu 4 Kohlenstoffatomen, Phenyl oder Benzyl bedeuten,
oder für Pyridyl oder Thienyl steht,

$R^2$ für einen geradkettigen, verzweigten, cyclischen, gesättigten oder ungesättigten Kohlenwasserstoffrest mit bis zu 6 Kohlenstoffatomen steht, der immer durch eine Gruppe $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$ substituiert ist,
worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3 oder 4 bedeutet,

d eine Zahl 0, 1, 2, 3 oder 4 bedeutet,

c die unten angegebene Bedeutung von a hat und mit dieser gleich oder verschieden ist,

$R^{15}$ und $R^{16}$ gleich oder verschieden sind und Phenyl bedeuten, das gegebenenfalls durch Fluor, Chlor, Nitro oder durch geradkettiges oder verzweigtes Alkyl oder Alkoxy mit jeweils bis zu 3 Kohlenstoffatomen substituiert ist,

$R^{19}$ und $R^{20}$ gleich oder verschieden sind und geradkettiges oder verzweigtes Alkyl mit 2 bis 8 Kohlenstoffatomen bedeuten, das durch Fluor, Chlor, Brom oder Phenyl substituiert ist, das seinerseits durch Methyl, Ethyl, Methoxy oder Ethoxy substituiert sein kann, oder
Cyclopropyl, Cyclopentyl oder Cyclohexyl bedeuten, oder
Phenyl bedeuten, das durch Fluor, Chlor, Brom, Nitro, Methyl, Ethyl, Methoxy oder Ethoxy substituiert ist,
oder

$R^{19}$ und $R^{20}$ gemeinsam mit dem Stickstoffatom einen 5- bis 6-gliedrigen Heterocyclus bilden, der gegebenenfalls durch ein Sauerstoff- oder Schwefelatom oder durch einen Rest der Formel $-NR^{21}$ unterbrochen ist,
worin

$R^{21}$ Wasserstoff oder geradkettiges oder verzweigtes Alkyl mit bis zu 10 Kohlenstoffatomen bedeutet, das gegebenenfalls durch Phenyl substituiert ist, das seinerseits durch Fluor, Chlor oder Brom substituiert sein kann, oder
Phenyl bedeutet, das gegebenenfalls durch Fluor, Chlor oder Brom substituiert ist,

oder der Kohlenwasserstoffrest substituiert ist durch Pyridyl, Tetrahydropyranyl, Furyl, Chromanyl, Piperazinyl, Piperidinyl, oder durch einen Rest der Formel

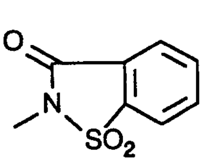

oder

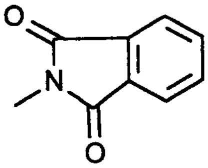

substituiert ist, die ihrerseits durch Benzyl substituiert sein können,

und wobei der Kohlenwasserstoffrest ($R^2$) gegebenenfalls zusätzlich durch Sauerstoff, Phenyliden oder durch eine Gruppe der Formel $-S(O)_a$ oder $-NR^6$ unterbrochen ist,

worin

a — eine Zahl 0, 1 oder 2 bedeutet

und

$R^6$ — Wasserstoff, Phenyl, Benzyl oder geradkettiges oder verzweigtes Alkyl mit bis zu 4 Kohlenstoffatomen oder Cyclopropyl, Cyclopentyl oder Cyclohexyl bedeutet,

und wobei der Kohlenwasserstoffrest gegebenenfalls durch Phenyl substituiert ist, das seinerseits durch Fluor, Chlor oder Brom substituiert sein kann, und

$R^3$ für Wasserstoff oder für geradkettiges oder verzweigtes Alkyl mit bis zu 3 Kohlenstoffatomen steht

und deren Salze.

Ganz besonders bevorzugt sind Verbindungen der allgemeinen Formel (I),

in welcher

$R^1$ — für Phenyl steht, das gegebenenfalls bis zu 2-fach gleich oder verschieden durch Fluor, Chlor, Cyano, Hydroxy, Nitro, Trifluormethyl, Methyl, Methoxy oder durch $-NR^4R^5$ substituiert ist,

worin

$R^4$ und $R^5$ — gleich oder verschieden sind und Wasserstoff oder geradkettiges oder verzweigtes Alkyl mit bis zu 4 Kohlenstoffatomen, Phenyl oder Benzyl bedeuten,

oder für Pyridyl oder Thienyl steht,

$R^2$ — für einen geradkettigen, verzweigten, cyclischen, gesättigten oder ungesättigten Kohlenwasserstoffrest mit bis zu 6 Kohlenstoffatomen steht,

der immer substituiert ist durch $-CO-NR^7R^8$, $-NR^9-CO-R^{10}$, $-NR^{11}-SO_2-R^{12}$,$-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$

worin

$R^7$, $R^8$, $R^9$, $R^{10}$, $R^{11}$, $R^{12}$, $R^{13}$, $R^{14}$, $R^{17}$ und $R^{18}$ — gleich oder verschieden sind und die unten angegebene Bedeutung von $R^6$ haben und mit dieser gleich oder verschieden sind,

b — eine Zahl 1, 2, 3 oder 4 bedeutet,

d — eine Zahl 0, 1, 2, 3 oder 4 bedeutet,

c — eine Zahl 0 oder 2 bedeutet,

$R^{15}$ und $R^{16}$ — Phenyl bedeuten,

$R^{19}$ und $R^{20}$ — gleich oder verschieden sind und geradkettiges oder verzweigtes Alkyl mit 2 bis 6 Kohlenstoffatomen bedeuten, das durch Fluor, Chlor oder Phenyl substituiert ist, das seinerseits durch Methyl oder Methoxy substituiert sein kann, oder

Cyclopropyl, Cyclopentyl oder Cyclohexyl bedeuten, oder

Phenyl bedeuten, das durch Fluor, Chlor, Methyl oder Methoxy substituiert ist,

oder

$R^{19}$ und $R^{20}$ gemeinsam mit dem Stickstoffatom einen 5- bis 6-gliedrigen Heterocyclus bilden, der gegebenenfalls durch ein Sauerstoff- oder Schwefelatom oder durch einen Rest der Formel $-NR^{21}$ unterbrochen ist,

worin

$R^{21}$ Wasserstoff oder geradkettiges oder verzweigtes Alkyl mit bis zu 4 Kohlenstoffatomen bedeutet, das gegebenenfalls durch Phenyl substituiert ist,

oder substituiert ist durch Pyridyl, Piperidyl oder durch einen Rest der Formel

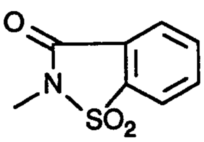

oder

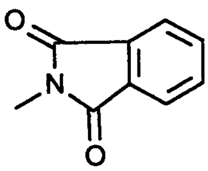

die ihrerseits durch Benzyl substituiert sein können, oder

durch Sauerstoff oder durch eine Gruppe der Formel $-NR^6$ unterbrochen ist,

worin

$R^6$ Wasserstoff, Phenyl, Benzyl oder geradkettiges oder verzweigtes Alkyl mit bis zu 4 Kohlenstoffatomen, Cyclopropyl, Cyclopentyl oder Cyclohexyl bedeutet, und wobei der Kohlenwasserstoffrest gegebenenfalls durch Phenyl substituiert ist, und

$R^3$ für Wasserstoff oder für Alkyl mit bis zu 2 Kohlenstoffatomen steht

und deren Salze.

Die Herstellung der erfindungsgemäßen Verbindungen der allgemeinen Formel (I) ist dadurch gekennzeichnet, daß man

[A] entweder Aldehyde der allgemeinen Formel (II)

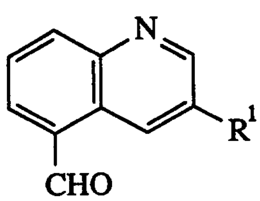

(II)

in welcher

$R^1$ die oben angegebene Bedeutung hat,

direkt mit Verbindungen der allgemeinen Formel (III)

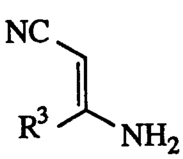

(III)

in welcher

$R^3$ die oben angegebene Bedeutung hat,

und Verbindungen der tautomeren Formel (IV) oder (IVa)

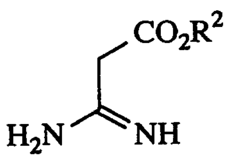 (IV) 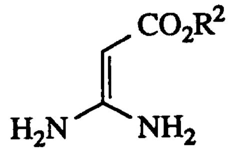 (IVa)

in welcher
$R^2$ die oben angegebene Bedeutung hat,
in inerten Lösemittteln bei Temperaturen zwischen 10 °C und 150 °C umsetzt,
oder
[B] Ylidenverbindungen der allgemeinen Formel (V)

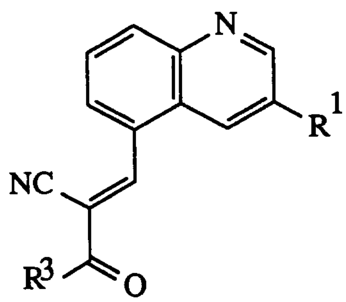 (V)

in welcher
$R^1$ und $R^3$ die oben angegebene Bedeutung haben,
mit Verbindungen der allgemeinen Formel (VI) bzw. (VIa)

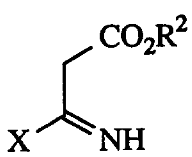 (VI) 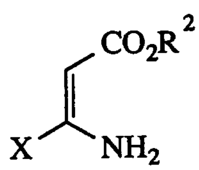 (VIa)

in welcher
$R^2$ die oben angegebene Bedeutung hat, und
X für die Aminogruppe oder für $C_1$-$C_4$-Alkoxy steht
gegebenenfalls in Anwesenheit von inerten organischen Lösemitteln bei Temperaturen von 10 °C bis 150 °C umsetzt, wobei für den Fall, daß X für $C_1$-$C_4$-Alkoxy steht, Ammoniumsalze, wie Ammoniumacetat zugegeben werden.

Im Fall der reinen Enantiomeren wird entweder das entstehende Diastereomerengemisch der jeweiligen Verbindungen der allgemeinen Formel (I), in welcher $R^2$ für einen definierten chiralen Rest steht, zunächst getrennt, dann in die entsprechenden Carbonsäuren ($R^2$ = H) überführt und in einem letzten Schritt verestert oder die jeweiligen Diastereomere werden direkt mit den entsprechenden Alkoholen, insbesondere in Form der Alkoholate umgeestert.

Die erfindungsgemäßen Verfahren können durch folgendes Formelschema beispielhaft erläutert werden:

[A]

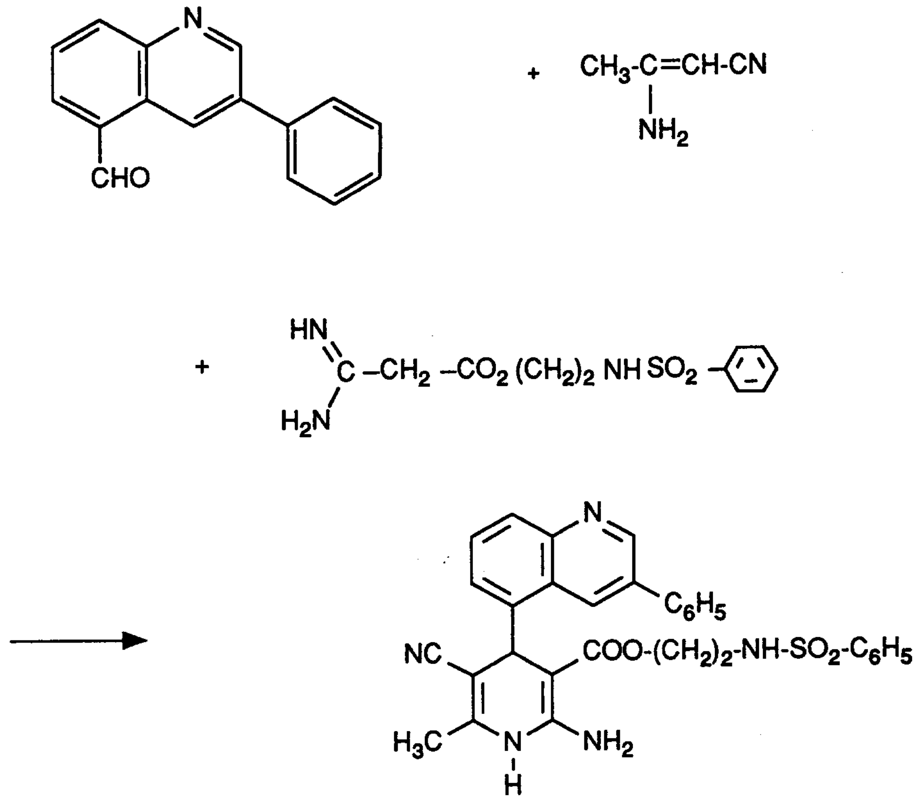

[B]

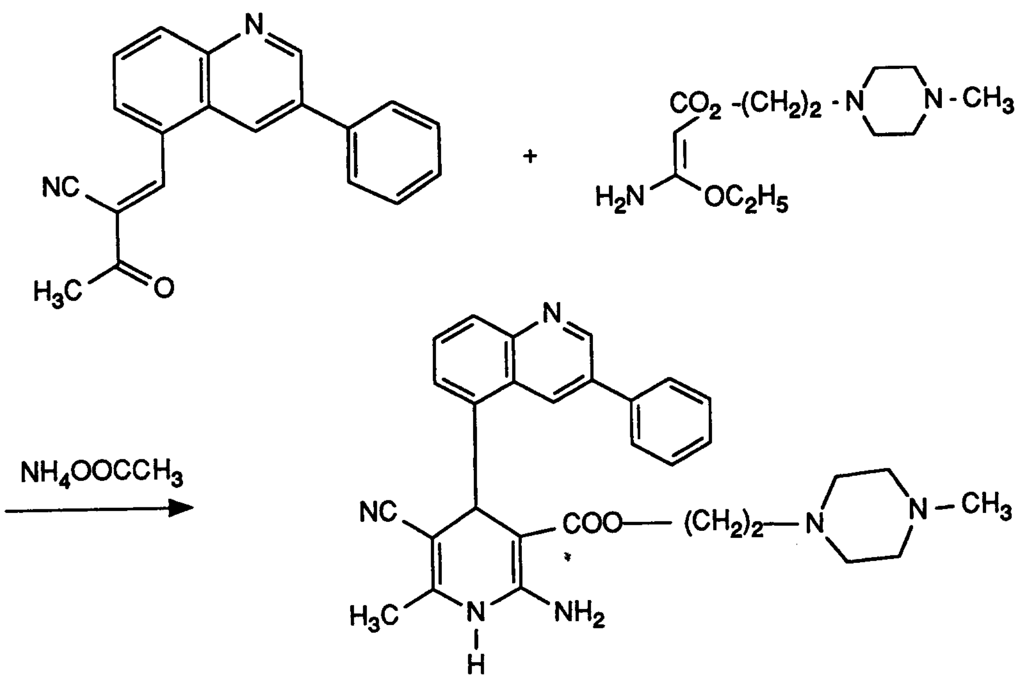


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A] oder [B] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind teilweise bekannt oder können nach üblichen Methoden hergestellt werden, indem man z.B. die entsprechenden Alkyl- oder Hydroxyalkyl-chinoline oxidiert oder die entsprechenden Carboxychinoline reduziert.

Alternativ kann auch 4-Amino-3-hydroxyphthalid, das durch übliche Hydrierung des literaturbekannten 4-Nitro-3-hydroxyphthalid in Anwesenheit eines Katalysators, bevorzugt mit Palladium/Bariumsulfat, erhalten wird, mit Verbindungen der allgemeinen Formel $R^1$-$CH_2$-CHO, die teilweise bekannt sind [vgl. z.B. Beilstein 7, 292], zu Verbindungen der allgemeinen Formel (II) über die entsprechenden Carbonsäuren umgesetzt werden.

Die Verbindungen der allgemeinen Formeln (III), (IV) und (IVa) sind bekannt oder können nach literaturbekannten Methoden hergestellt werden.

Die Ylidenverbindungen der allgemeinen Formel (V) sind teilweise bekannt oder können hergestellt werden, indem man Verbindungen der allgemeinen Formel (VII)

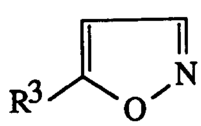

(VII)

in welcher

$R^3$ die oben angegebene Bedeutung hat,

mit Alkalihydroxiden oder Alkalialkoholaten in die Alkalisalze der Verbindungen der allgemeinen Formel (VIII)

$R^3$-CO-$CH_2$-CN (VIII)

in welcher

$R^3$ die oben angegebene Bedeutung hat,

überführt,

und diese entweder in situ oder nach Isolation, mit Aldehyden der allgemeinen Formel (II) in einem der oben aufgeführten inerten Lösemitteln, vorzugsweise in Alkoholen, Essigester, Methylenchlorid, Acetonitril, Chloroform oder Ethern, unter Zusatz von Säure, vorzugsweise Essigsäure und gegebenenfalls in Anwesenheit eines Katalysators, beispielsweise Piperidin-acetat bei Temperaturen zwischen 0°C und 150°C, vorzugsweise zwischen 20°C und 110°C umsetzt.

Die Verbindungen der allgemeinen Formeln (VI), (VIa), (VII) und (VIII) sind bekannt oder können nach üblichen Methoden hergestellt werden.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Vorzugsweise zeigen sie eine positiv inotrope Wirkung. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur

Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

2-Amino-5-cyano-6-methyl-4-(3-phenylchinolin-5-yl)-1,4-dihydropyridin-3-carbonsäure-1S,2R-(2-benzyloxycarbonylamino-1-phenyl)-propylester

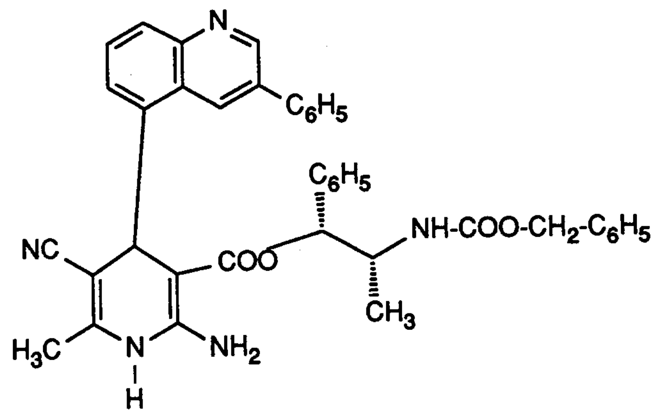

1,5 g (5 mmol) 2-(3-Phenylchinolin-5-yliden)-3-oxo-buttersäurenitril werden in 10 ml Isopropanol mit 2 g (5 mmol) 3-Ethoxy-3-imino-propionsäure-1S,2R-(2-benzyloxycarbonylamino-1-phenyl)-propylester und 770 mg Ammoniumacetat 18 Stunden zum Rückfluß erhitzt. Es wird abgekühlt und eingeengt. Es wird in Essigester / Wasser gelöst, getrennt, die Essigesterphase mit Wasser gewaschen, getrocknet und eingeengt. Die beiden Diastereomeren werden mit Dichlormethan / Essigester-Gemischen über eine Kieselgelsäule getrennt. Man erhält 514 mg des Diastereomeren A vom Schmelzpunkt 159-163 °C.
$R_f$-Wert = 0,6, DC-Alufolio Merck, Dichlormethan / Essigester = 1:1
Diastereomer B: Schmelzpunkt: ab 160 °C
$R_f$-Wert = 0,48
In Analogie zur Vorschrift aus Beispiel 1 werden die in Tabelle 1 aufgeführten Verbindungen hergestellt:

<u>Tabelle 1</u>:

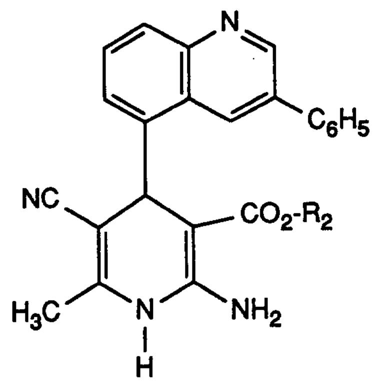

| Bsp.-Nr. | $R^2$ | F°C | Diastereomer | Enantiomer |
|---|---|---|---|---|
| 2 | 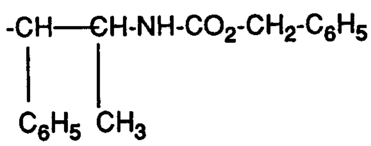 | >159°C | A | |
| 3 | 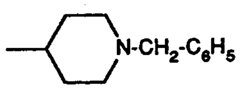 | 148-150 | | (-) |
| 4 | 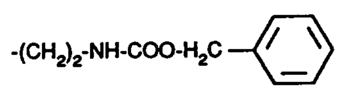 | 154-155 | | |

**Patentansprüche**

**1.** 2-Amino-5-cyano-4-chinolyldihydropyridinester der allgemeinen Formel (I)

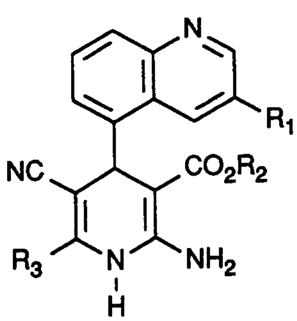

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Hydroxy, Trifluormethyl, Trifluormethoxy, Trifluormethylthio,
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel $-NR^4R^5$ substituiert ist,

worin

$R^4$ und $R^5$ gleich oder verschieden sind und Wasserstoff, geradkettiges oder verzweigtes Alkyl mit bis zu 8 Kohlenstoffatomen, Phenyl oder Benzyl bedeuten,

oder

für Pyridyl oder Thienyl steht, die gegebenenfalls durch Halogen substituiert sind,

$R^2$ für einen geradkettigen, verzweigten, cyclischen, gesättigten oder ungesättigten Kohlenwasserstoffrest mit bis zu 12 Kohlenstoffatomen steht, der immer 1- oder 2-fach gleich oder verschieden substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$

worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3, 4 oder 5 bedeutet,

d eine Zahl 0, 1, 2, 3, 4 oder 5 bedeutet,

c die unten angegebene Bedeutung von a hat und mit dieser gleich oder verschieden ist,

$R^{15}$ und $R^{16}$ gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert ist,

$R^{19}$ und $R^{20}$ gleich oder verschieden sind und geradkettiges oder verzweigtes Alkyl mit 2 bis 8 Kohlenstoffatomen bedeuten, das durch Halogen, Hydroxy oder Phenyl substituiert ist, das seinerseits durch Halogen, Nitro, Hydroxy oder durch geradkettiges oder verzweigtes Alkyl oder Alkoxy mit jeweils bis zu 4 Kohlenstoffatomen substituiert sein kann,

oder

Cycloalkyl mit 3 bis 8 Kohlenstoffatomen bedeuten, oder geradkettiges oder verzweigtes Alkenyl mit 3 bis 8 Kohlenstoffatomen bedeuten, oder

Phenyl bedeuten, das durch Halogen, Nitro oder geradkettiges oder verzweigtes Alkyl oder Alkoxy mit bis zu 4 Kohlenstoffatomen substituiert ist,

oder

$R^{19}$ und $R^{20}$ gemeinsam mit dem Stickstoffatom einen 4- bis 7-gliedrigen Heterocyclus bilden, der gegebenenfalls durch ein Sauerstoff- oder Schwefelatom oder durch einen Rest der Formel $-NR^{21}$ unterbrochen ist,

worin

$R^{21}$ Wasserstoff oder geradkettiges oder verzweigtes Alkyl mit bis zu 8 Kohlenstoffatomen bedeutet, das gegebenenfalls durch Phenyl substituiert ist, das seinerseits durch Halogen substituiert sein kann, oder

Phenyl bedeutet, das gegebenenfalls durch Halogen substituiert ist,

oder substituiert ist durch einen 3- bis 7-gliedrigen gesättigten oder ungesättigten Heterocyclus oder Heterocyclyloxyring mit bis zu 3 Heteroatomen aus der Reihe S, N oder O, der seinerseits bis zu

2-fach gleich oder verschieden durch Halogen, Hydroxy oder durch Aryl oder Arylsulfonyl mit 6 bis 10 Kohlenstoffatomen substituiert sein kann, wobei die Cyclen ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,

oder der Heterocyclus gegebenenfalls bis zu 2-fach gleich oder verschieden durch geradkettiges oder verzweigtes Alkyl oder Alkenyl mit jeweils bis zu 8 Kohlenstoffatomen substituiert ist, die ihrerseits bis zu 2-fach gleich oder verschieden durch $-NR^{22}R^{23}$ substituiert sein können,

worin

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, das gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können, oder

Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

oder

$R^{22}$ und $R^{23}$ gemeinsam mit dem Stickstoffatom einen 5- bis 7-gliedrigen, gesättigen oder ungesättigten Heterocyclus mit bis zu 3 Heteroatomen aus der Reihe S, N oder O bilden, oder Alkyl oder Alkenyl ihrerseits durch Phenyl oder Phenoxy substituiert sein können, die gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sind,

der gegebenenfalls durch ein Sauerstoffatom oder durch Aryliden mit 6 bis 10 Kohlenstoffatomen unterbrochen sein kann, das gegebenenfalls durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

oder der gegebenenfalls durch eine Gruppe der Formel $-S(O)_a$ oder $-NR^6$ unterbrochen ist,

worin

a eine Zahl 0, 1 oder 2 bedeutet

und

$R^6$ Wasserstoff, Aryl mit 6 bis 10 Kohlenstoffatomen oder geradkettiges oder verzweigtes Alkyl mit bis zu 6 Kohlenstoffatomen oder Cycloalkyl mit 3 bis 8 Kohlenstoffatomen bedeutet, wobei Alkyl und Cycloalkyl gegebenenfalls durch Aryl mit 6 bis 10 Kohlenstoffatomen substituiert sind

und wobei der Kohlenwasserstoffrest gegebenenfalls zusätzlich durch Phenyl substituiert ist, das seinerseits durch Halogen substituiert sein kann,

und

$R^3$ für Wasserstoff oder für geradkettiges oder verzweigtes Alkyl mit bis zu 8 Kohlenstoffatomen steht,

und deren Salze.

**2.** Verbindungen der allgemeinen Formel (I) gemäß Anspruch 1

in welcher

$R^1$ für Phenyl steht, das gegebenenfalls bis zu 2-fach gleich oder verschieden durch Halogen, Nitro, Cyano, Hydroxy, Trifluormethyl oder durch geradkettiges oder verzweigtes Alkyl oder Alkoxy mit jeweils bis zu 4 Kohlenstoffatomen oder durch eine Gruppe der Formel $-NR^4R^5$ substituiert ist,

worin

$R^4$ und $R^5$ gleich oder verschieden sind und Wasserstoff, geradkettiges oder verzweigtes Alkyl mit bis zu 6

Kohlenstoffatomen, Phenyl oder Benzyl bedeuten,
oder für Pyridyl oder Thienyl steht, die gegebenenfalls durch Fluor, Chlor oder Brom substituiert sind,

$R^2$ für einen geradkettigen, verzweigten, cyclischen, gesättigten oder ungesättigten Kohlenwasserstoffrest mit bis zu 10 Kohlenstoffatomen steht, der immer 1- oder 2-fach gleich oder verschieden substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$

worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3 oder 4 bedeutet,

d eine Zahl 0, 1, 2, 3 oder 4 bedeutet,

c die unten angegebene Bedeutung von a hat und mit dieser gleich oder verschieden ist,

$R^{15}$ und $R^{16}$ gleich oder verschieden sind und Phenyl bedeuten, das gegebenenfalls bis zu 2-fach gleich oder verschieden durch Fluor, Chlor, Nitro, Carboxy oder durch geradkettiges oder verzweigtes Alkyl, Alkoxy oder Alkoxycarbonyl mit jeweils bis zu 4 Kohlenstoffatomen substituiert ist,

$R^{19}$ und $R^{20}$ gleich oder verschieden sind und geradkettiges oder verzweigtes Alkyl mit 2 bis 8 Kohlenstoffatomen bedeuten, das durch Fluor, Chlor, Brom oder Phenyl substituiert ist, das seinerseits durch Fluor, Chlor, Brom, Methyl, Ethyl, Methoxy oder Ethoxy substituiert sein kann, oder

Cyclopropyl, Cyclopentyl oder Cyclohexyl bedeuten, oder Phenyl bedeuten, das durch Fluor, Chlor, Brom, Nitro, Methyl, Ethyl, Methoxy oder Ethoxy substituiert ist,

oder

$R^{19}$ und $R^{20}$ gemeinsam mit dem Stickstoffatom einen 5- bis 7-gliedrigen Heterocyclus bilden, der gegebenenfalls durch ein Sauerstoff- oder Schwefelatom oder durch einen Rest der Formel $-NR^{21}$ unterbrochen ist,

worin

$R^{21}$ Wasserstoff oder geradkettiges oder verzweigtes Alkyl mit bis zu 10 Kohlenstoffatomen bedeutet, das gegebenenfalls durch Phenyl substituiert ist, das seinerseits durch Fluor, Chlor oder Brom substituiert sein kann, oder

Phenyl bedeutet, das gegebenenfalls durch Fluor, Chlor oder Brom substituiert ist,

oder substituiert ist durch Pyridyl, Tetrahydropyranyl, Pyrazolyl, Furyl, Chromanyl, Piperazinyl, Piperidinyl, Isochinolidinyl oder durch einen Rest der Formel

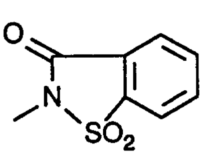 , 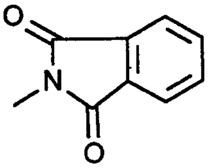 oder 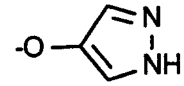

die ihrerseits durch Fluor, Chlor, Benzyl, Phenyl oder Phenylsulfonyl substituiert sein können,

und wobei der Kohlenwasserstoffrest ($R^2$) gegebenenfalls zusätzlich durch Sauerstoff, durch Phenyliden oder durch $-S(O)_a$ oder $-NR^6$ unterbrochen ist,

worin

a eine Zahl 0, 1 oder 2 bedeutet und

$R^6$ Wasserstoff, Phenyl oder geradkettiges oder verzweigtes Alkyl mit bis zu 5 Kohlenstoffatomen, Cyclopropyl, Cyclopentyl oder Cyclohexyl bedeutet, die gegebenenfalls durch Phenyl substituiert sind

und wobei der Kohlenwasserstoffrest gegebenenfalls durch Phenyl substituiert ist, das seinerseits durch Fluor, Chlor oder Brom substituiert sein kann,

$R^3$ für Wasserstoff oder für geradkettiges oder verzweigtes Alkyl mit bis zu 4 Kohlenstoffatomen steht

und deren Salze.

**3.** Verbindungen der allgemeinen Formel (I) gemäß Anspruch 1

in welcher

| | |
|---|---|
| $R^1$ | für Phenyl steht, das gegebenenfalls bis zu 2-fach gleich oder verschieden durch Fluor, Chlor, Nitro, Cyano, Hydroxy, Trifluormethyl, Methyl, Ethyl, Methoxy oder Ethoxy oder durch eine Gruppe der Formel $-NR^4R^5$ substituiert ist, worin |
| $R^4$ und $R^5$ | gleich oder verschieden sind und Wasserstoff oder geradkettiges oder verzweigtes Alkyl mit bis zu 4 Kohlenstoffatomen, Phenyl oder Benzyl bedeuten, |
| | oder für Pyridyl oder Thienyl steht, |
| $R^2$ | für einen geradkettigen, verzweigten, cyclischen, gesättigten oder ungesättigten Kohlenwasserstoffrest mit bis zu 6 Kohlenstoffatomen steht, der immer durch eine Gruppe $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$ substituiert ist, worin |
| $R^7$, $R^8$, $R^9$, $R^{10}$, $R^{11}$, $R^{12}$, $R^{13}$, $R^{14}$, $R^{17}$ und $R^{18}$ | gleich oder verschieden sind und die unten angegebene Bedeutung von $R^6$ haben und mit dieser gleich oder verschieden sind |
| b | eine Zahl 1, 2, 3 oder 4 bedeutet, |
| d | eine Zahl 0, 1, 2, 3 oder 4 bedeutet, |
| c | die unten angegebene Bedeutung von a hat und mit dieser gleich oder verschieden ist, |
| $R^{15}$ und $R^{16}$ | gleich oder verschieden sind und Phenyl bedeuten, das gegebenenfalls durch Fluor, Chlor, Nitro oder durch geradkettiges oder verzweigtes Alkyl oder Alkoxy mit jeweils bis zu 3 Kohlenstoffatomen substituiert ist, |
| $R^{19}$ und $R^{20}$ | gleich oder verschieden sind und geradkettiges oder verzweigtes Alkyl mit 2 bis 8 Kohlenstoffatomen bedeuten, das durch Fluor, Chlor, Brom oder |

Phenyl substituiert ist, das seinerseits durch Methyl, Ethyl, Methoxy oder Ethoxy substituiert sein kann, oder
Cyclopropyl, Cyclopentyl oder Cyclohexyl bedeuten, oder Phenyl bedeuten, das durch Fluor, Chlor, Brom, Nitro, Methyl, Ethyl, Methoxy oder Ethoxy substituiert ist,
oder

$R^{19}$ und $R^{20}$ gemeinsam mit dem Stickstoffatom einen 5- bis 6-gliedrigen Heterocyclus bilden, der gegebenenfalls durch ein Sauerstoff- oder Schwefelatom oder durch einen Rest der Formel $-NR^{21}$ unterbrochen ist,
worin

$R^{21}$ Wasserstoff oder geradkettiges oder verzweigtes Alkyl mit bis zu 10 Kohlenstoffatomen bedeutet, das gegebenenfalls durch Phenyl substituiert ist, das seinerseits durch Fluor, Chlor oder Brom substituiert sein kann, oder
Phenyl bedeutet, das gegebenenfalls durch Fluor, Chlor oder Brom substituiert ist,

oder der Kohlenwasserstoffrest substituiert ist durch Pyridyl, Tetrahydropyranyl, Furyl, Chromanyl, Piperazinyl, Piperidinyl, oder durch einen Rest der Formel

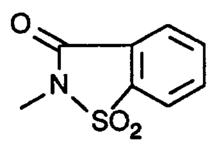

oder

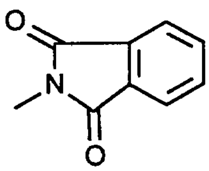

substituiert ist, die ihrerseits durch Benzyl substituiert sein können,

und wobei der Kohlenwasserstoffrest ($R^2$) gegebenenfalls zusätzlich durch Sauerstoff, Phenyliden oder durch eine Gruppe der Formel $-S(O)_a$ oder $-NR^6$ unterbrochen ist,

worin

a eine Zahl 0, 1 oder 2 bedeutet
und

$R^6$ Wasserstoff, Phenyl, Benzyl oder geradkettiges oder verzweigtes Alkyl mit bis zu 4 Kohlenstoffatomen oder Cyclopropyl, Cyclopentyl oder Cyclohexyl bedeutet,
und wobei der Kohlenwasserstoffrest gegebenenfalls durch Phenyl substituiert ist, das seinerseits durch Fluor, Chlor oder Brom substituiert sein kann, und

$R^3$ für Wasserstoff oder für geradkettiges oder verzweigtes Alkyl mit bis zu 3 Kohlenstoffatomen steht

und deren Salze.

**4.** Verbindungen der allgemeinen Formel (I) gemäß Anspruch 1
in welcher

$R^1$ für Phenyl steht, das gegebenenfalls bis zu 2-fach gleich oder verschieden durch Fluor, Chlor, Cyano, Hydroxy, Nitro, Trifluormethyl, Methyl, Methoxy oder durch $-NR^4R^5$ substituiert ist,
worin

$R^4$ und $R^5$ gleich oder verschieden sind und Wasserstoff oder geradkettiges oder verzweigtes Alkyl mit bis zu 4 Kohlenstoffatomen, Phenyl oder Benzyl bedeuten,
oder für Pyridyl oder Thienyl steht,

$R^2$ für einen geradkettigen, verzweigten, cyclischen, gesättigten oder ungesättigten Kohlenwasserstoffrest mit bis zu 6 Kohlenstoffatomen steht,

der immer substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$

worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3 oder 4 bedeutet,

d eine Zahl 0, 1, 2, 3 oder 4 bedeutet,

c eine Zahl 0 oder 2 bedeutet,

$R^{15}$ und $R^{16}$ Phenyl bedeuten,

$R^{19}$ und $R^{20}$ gleich oder verschieden sind und geradkettiges oder verzweigtes Alkyl mit 2 bis 6 Kohlenstoffatomen bedeuten, das durch Fluor, Chlor oder Phenyl substituiert ist, das seinerseits durch Methyl oder Methoxy substituiert sein kann, oder Cyclopropyl, Cyclopentyl oder Cyclohexyl bedeuten, oder Phenyl bedeuten, das durch Fluor, Chlor, Methyl oder Methoxy substituiert ist,

oder

$R^{19}$ und $R^{20}$ gemeinsam mit dem Stickstoffatom einen 5- bis 6-gliedrigen Heterocyclus bilden, der gegebenenfalls durch ein Sauerstoff- oder Schwefelatom oder durch einen Rest der Formel $-NR^{21}$ unterbrochen ist,

worin

$R^{21}$ Wasserstoff oder geradkettiges oder verzweigtes Alkyl mit bis zu 4 Kohlenstoffatomen bedeutet, das gegebenenfalls durch Phenyl substituiert ist,

oder substituiert ist durch Pyridyl, Piperidyl oder durch einen Rest der Formel

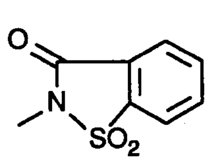

oder

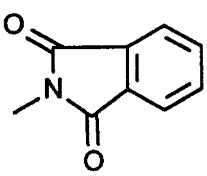

die ihrerseits durch Benzyl substituiert sein können, oder

durch Sauerstoff oder durch eine Gruppe der Formel $-NR^6$ unterbrochen ist,

worin

$R^6$ Wasserstoff, Phenyl, Benzyl oder geradkettiges oder verzweigtes Alkyl mit bis zu 4 Kohlenstoffatomen, Cyclopropyl, Cyclopentyl oder Cyclohexyl bedeutet,

und wobei der Kohlenwasserstoffrest gegebenenfalls durch Phenyl substituiert ist, und

$R^3$ für Wasserstoff oder für Alkyl mit bis zu 2 Kohlenstoffatomen steht

und deren Salze.

**5.** Verfahren zur Herstellung von Verbindungen der allgemeinen Formel (I)

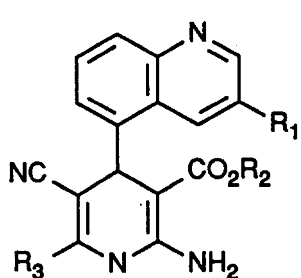

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Hydroxy, Trifluormethyl, Trifluormethoxy, Trifluormethylthio, oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder durch geradkettiges oder verzweigtes Alkoxy oder Alkoxycarbonyl mit jeweils bis zu 8 Kohlenstoffatomen, Carboxy, Amino oder durch eine Gruppe der Formel $-NR^4R^5$ substituiert ist,
worin

$R^4$ und $R^5$ gleich oder verschieden sind und Wasserstoff, geradkettiges oder verzweigtes Alkyl mit bis zu 8 Kohlenstoffatomen, Phenyl oder Benzyl bedeuten,
oder
für Pyridyl oder Thienyl steht, die gegebenenfalls durch Halogen substituiert sind,

$R^2$ für einen geradkettigen, verzweigten, cyclischen, gesättigten oder ungesättigten Kohlenwasserstoffrest mit bis zu 12 Kohlenstoffatomen steht, der immer 1- oder 2-fach gleich oder verschieden substituiert ist durch $-CO-NR^7R^8$, $-NR^9-CO-R^{10}$, $-NR^{11}-SO_2-R^{12}$, $-SO_2-NR^{13}R^{14}$, $O-NO_2$, $-O-(CH_2)_b-R^{15}$, $-S(O)_c-(CH_2)_d-R^{16}$, $-NR^{17}-COOR^{18}$ oder $-NR^{19}R^{20}$
worin

$R^7$, $R^8$, $R^9$, $R^{10}$, $R^{11}$, $R^{12}$, $R^{13}$, $R^{14}$, $R^{17}$ und $R^{18}$ gleich oder verschieden sind und die unten angegebene Bedeutung von $R^6$ haben und mit dieser gleich oder verschieden sind,

b eine Zahl 1, 2, 3, 4 oder 5 bedeutet,

d eine Zahl 0, 1, 2, 3, 4 oder 5 bedeutet,

c die unten angegebene Bedeutung von a hat und mit dieser gleich oder verschieden ist,

$R^{15}$ und $R^{16}$ gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert ist,

$R^{19}$ und $R^{20}$ gleich oder verschieden sind und geradkettiges oder verzweigtes Alkyl mit 2 bis 8 Kohlenstoffatomen bedeuten, das durch Halogen, Hydroxy oder Phenyl substituiert ist, das seinerseits durch Halogen, Nitro, Hydroxy oder durch geradkettiges oder verzweigtes Alkyl oder Alkoxy mit jeweils bis zu 4 Kohlenstoffatomen substituiert sein kann,
oder
Cycloalkyl mit 3 bis 8 Kohlenstoffatomen bedeuten, oder geradkettiges oder verzweigtes Alkenyl mit 3 bis 8 Kohlenstoffatomen bedeuten, oder
Phenyl bedeuten, das durch Halogen, Nitro oder geradkettiges oder verzweigtes Alkyl oder Alkoxy mit bis zu 4 Kohlenstoffatomen substituiert ist,
oder

$R^{19}$ und $R^{20}$ gemeinsam mit dem Stickstoffatom einen 4- bis

7-gliedrigen Heterocyclus bilden, der gegebenenfalls durch ein Sauerstoff- oder Schwefelatom oder durch einen Rest der Formel $-NR^{21}$ unterbrochen ist,
worin

$R^{21}$ Wasserstoff oder geradkettiges oder verzweigtes Alkyl mit bis zu 8 Kohlenstoffatomen bedeutet, das gegebenenfalls durch Phenyl substituiert ist, das seinerseits durch Halogen substituiert sein kann, oder
Phenyl bedeutet, das gegebenenfalls durch Halogen substituiert ist,

oder substituiert ist durch einen 3- bis 7-gliedrigen gesättigten oder ungesättigten Heterocyclus oder Heterocyclyloxyring mit bis zu 3 Heteroatomen aus der Reihe S, N oder O, der seinerseits bis zu 2-fach gleich oder verschieden durch Halogen, Hydroxy oder durch Aryl oder Arylsulfonyl mit 6 bis 10 Kohlenstoffatomen substituiert sein kann, wobei die Cyclen ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,

oder der Heterocyclus gegebenenfalls bis zu 2-fach gleich oder verschieden durch geradkettiges oder verzweigtes Alkyl oder Alkenyl mit jeweils bis zu 8 Kohlenstoffatomen substituiert ist, die ihrerseits bis zu 2-fach gleich oder verschieden durch $-NR^{22}R^{23}$ substituiert sein können,
worin

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, das gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können, oder Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,
oder

$R^{22}$ und $R^{23}$ gemeinsam mit dem Stickstoffatom einen 5- bis 7-gliedrigen, gesättigen oder ungesättigten Heterocyclus mit bis zu 3 Heteroatomen aus der Reihe S, N oder O bilden,

oder Alkyl oder Alkenyl ihrerseits durch Phenyl oder Phenoxy substituiert sein können, die gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sind,

der gegebenenfalls durch ein Sauerstoffatom oder durch Aryliden mit 6 bis 10 Kohlenstoffatomen unterbrochen sein kann, das gegebenenfalls durch Halogen, Nitro, Amino, Hydroxy, Carboxy oder durch geradkettiges oder verzweigtes Alkyl, Alkoxy oder Alkoxycarbonyl mit jeweils bis zu Kohlenstoffatomen substituiert ist,

oder der gegebenenfalls durch eine Gruppe der Formel $-S(O)_a$ oder $-NR^6$ unterbrochen ist,
worin

a eine Zahl 0, 1 oder 2 bedeutet
und

$R^6$ Wasserstoff, Aryl mit 6 bis 10 Kohlenstoffatomen oder geradkettiges oder verzweigtes Alkyl mit bis zu 6 Kohlenstoffatomen oder Cycloalkyl mit 3 bis 8 Kohlenstoffatomen bedeutet, wobei Alkyl und Cycloalkyl gegebenenfalls durch Aryl mit 6 bis 10 Kohlenstoffatomen substituiert sind

und wobei der Kohlenwasserstoffrest gegebenenfalls zusätzlich durch Phenyl substituiert ist, das seinerseits durch Halogen substituiert sein kann,
und

$R^3$ für Wasserstoff oder für geradkettiges oder verzweigtes Alkyl mit bis zu 8 Kohlenstoffatomen

steht,

und deren Salze, dadurch gekennzeichnet, daß man

[A] entweder Aldehyde der allgemeinen Formel (II)

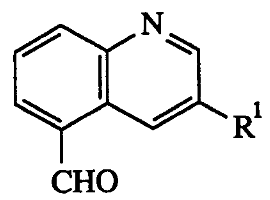

(II)

in welcher

$R^1$ die oben angegebene Bedeutung hat,

direkt mit Verbindungen der allgemeinen Formel (III)

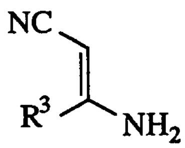

(III)

in welcher

$R^3$ die oben angegebene Bedeutung hat,

und Verbindungen der tautomeren Formel (IV) oder (IVa)

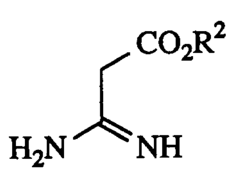

(IV)

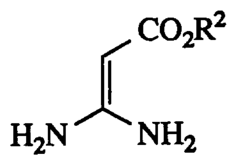

(IVa)

in welcher

$R^2$ die oben angegebene Bedeutung hat,

in inerten Lösemittteln bei Temperaturen zwischen 10 ° C und 150 ° C umsetzt,

oder

[B] Ylidenverbindungen der allgemeinen Formel (V)

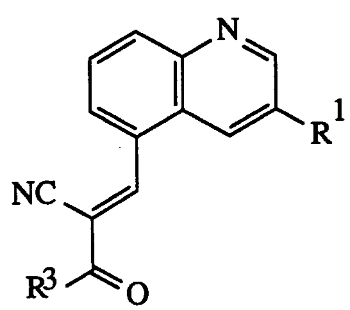

(V) .

in welcher

$R^1$ und $R^3$ die oben angegebene Bedeutung haben,

mit Verbindungen der allgemeinen Formel (VI) bzw. (VIa)

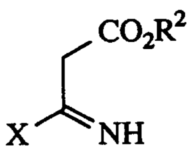

(VI)

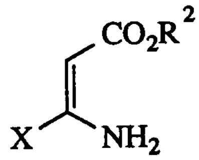

(VIa)

in welcher
$R^2$ die oben angegebene Bedeutung hat, und
X für die Aminogruppe oder für $C_1$-$C_4$-Alkoxy steht

gegebenenfalls in Anwesenheit von inerten organischen Lösemitteln bei Temperaturen von 10°C bis 150°C umsetzt, wobei für den Fall, daß X für $C_1$-$C_4$-Alkoxy steht, Ammoniumsalze, zugegeben werden.

6. Verbindungen der allgmeinen Formel (I) gemäß Anspruch 1 zur Verwendung bei der Behandlung von Herz-Kreislauferkrankungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

9. Verwendung von Verbindungen der allgemeinen Formel (I) gemäß Anspruch 1 in Arzneimitteln mit positiv inotroper Wirkung.